# EUROPÄISCHE PATENTANMELDUNG

(11) **EP 2 165 717 A1**
(43) Veröffentlichungstag der Anmeldung: **24.03.2010**
(21) Anmeldenummer: 09007797.5
(22) Anmeldetag: 12.06.2009
(51) Int. Cl.: A61L 2/00, C07K 14/435, C12N 9/94

(54) **Verfahren zur Verringerung der viralen und mikrobiellen Belastung feststoffhaltiger biologischer Extrakte**

(30) Priorität: 27.08.2008 DE 102008039860
(71) Anmelder: Nordmark Arzneimittel GmbH & Co.KG, 25436 Uetersen (DE)
(72) Erfinder: Rämsch, Christian, Dr., 25436 Gross Nordende (DE); Schräder, Thomas, Dr., 25436 Uetersen (DE); Moest, Thomas, Dr., 25436 Moorrege (DE); Kurfürst, Manfred, Dr., 25436 Moorrege (DE)
(74) Vertreter: Richter, Werdermann, Gerbaulet & Hofmann

(57) **Zusammenfassung**

Die Erfindung betrifft ein Verfahren zur Verringerung der viralen und mikrobiellen Belastung feststoffhaltiger biologischer Extrakte. Dabei ist vorgesehen, dass das Verfahren die Schritte
(a) Bereitstellen des feststoffhaltigen biologischen Extraktes, umfassend zumindest einen biologisch aktiven Wirkstoff, ausgewählt aus Enzymen, Proteinen und Peptiden, oder ein Gemisch derartiger Wirkstoffe; und
(b) Unterziehen des in Schritt (a) bereitgestellten Extraktes einer Hochdruckbehandlung;

umfasst, wobei
- die biologische Aktivität des feststoffhaltigen biologischen Extraktes nach der Hochdruckbehandlung zumindest 50 % der biologischen Aktivität des feststoffhaltigen biologischen Extraktes vor der Hochdruckbehandlung entspricht; und
- die virale Infektiosität des feststoffhaltigen biologischen Extraktes nach der Hochdruckbehandlung um einen Faktor von größer als 1 log₁₀ im Vergleich zur viralen Infektiosität vor der Hochdruckbehandlung verringert worden ist.

## Beschreibung

Die Erfindung betrifft ein Verfahren zur Verringerung der viralen und mikrobiellen Belastung feststoffhaltiger biologischer Extrakte, ein mittels dieses Verfahrens hergestelltes feststoffhaltiges biologisches Extrakt, sowie Verwendungen eines solchen feststoffhaltigen biologischen Extraktes.

Extrakte, die aus biologischem Ausgangsmaterial gewonnen werden, können eine hohe Virenbelastung aufweisen. Viren sind Nukleinsäuren, die von einer Proteinhülle umgeben sind. Bei behüllten Viren kommt noch eine äußere Lipidhülle hinzu. Da sich Viren nicht eigenständig replizieren können, sind sie auf Wirte angewiesen. Dem entsprechend kommen sie in praktisch allen Lebewesen der Erde vor. Die wenigsten der bekannten Viren sind für den Menschen pathogen, da sie eine hohe Wirtsspezifität besitzen. Um eine Gefährdung der Konsumenten von vorn herein auszuschließen, sollten Extrakte, die für den menschlichen Verzehr bestimmt sind oder die als Wirkstoff in Arzneimitteln eingesetzt werden, grundsätzlich eine möglichst geringe bzw. keine Virenbelastung aufweisen. Nicht immer führt das eigentliche Produktionsverfahren zu einer signifikanten Inaktivierung oder Entfernung der vorhandenen Viren, so dass, insbesondere bei der Herstellung pharmazeutischer Wirkstoffe, zusätzliche Abreicherungs- oder Inaktivierungsschritte in das Verfahren integriert werden müssen.

Für die Abreicherung oder Inaktivierung von Viren und Mikroorganismen sind zahlreiche Methoden beschrieben [K.H. Wallhäuser, Praxis der Sterilisation Desinfektion Konservierung, Thieme Verlag, Stuttgart 1995]. Neben der mechanischen Entfernung durch z. B. Chromatographie oder Filtration können diese Kontaminationen durch Zusatz chemischer Verbindungen selektiv inaktiviert werden. Letzteres Verfahren ist aber in sofern problematisch, als diese Verbindungen wieder vollständig entfernt werden müssen, damit sie im Endprodukt keine toxischen Effekte hervorrufen. Physikalische Methoden, wie z. B. Hitzebehandlung oder Bestrahlung sind ebenfalls gängige Verfahren zur Inaktivierung von Viren oder Mikroorganismen.

Eine besondere Herausforderung ist die Inaktivierung oder Entfernung von Viren aus komplexen biologischen Extrakten, deren Wirksubstanz Enzymgemische sind, ohne dabei die enzymatische Aktivität der Proteine zu zerstören oder zu verändern.

Von besonderem wirtschaftlichem Interesse ist der pharmazeutische Wirkstoff Pankreatin, der als Extrakt aus der Schweinepankreas gewonnen wird und in getrockneter Form als orales Therapeutikum Anwendung findet, wie in DE 3248588 A1 beschrieben.

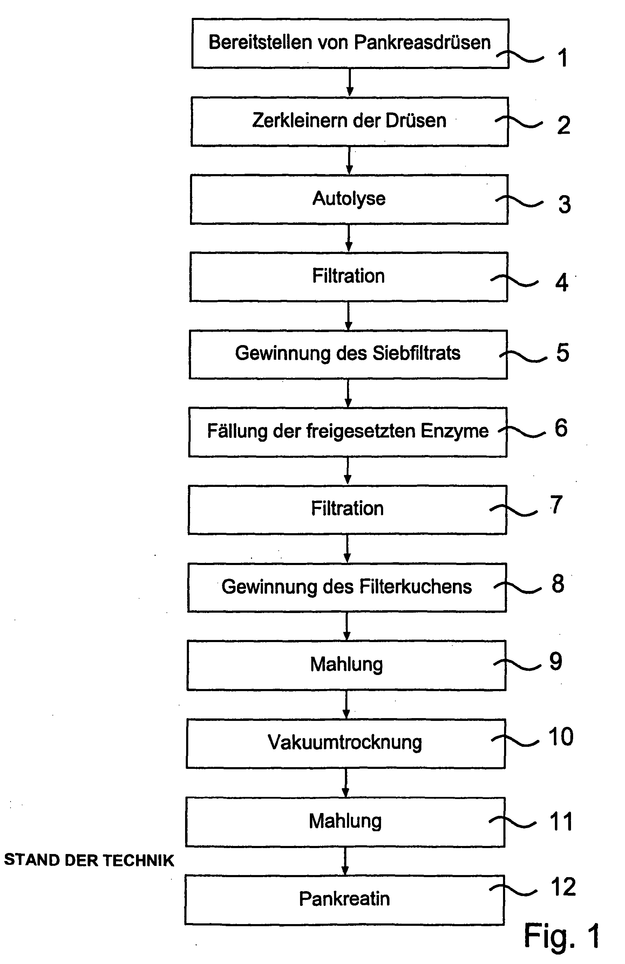

Ein typisches Verfahren zur Herstellung von Pankreatin wird nachstehend unter Bezugnahme auf Fig. 1 beschrieben. Die von Hausschweinen stammenden Pankreasdrüsen 1 werden zunächst zerkleinert 2 und einer Autolyse 3 unterzogen. Durch Filtration 4 des so erhaltenen Zwischenproduktes wird das Siebfiltrat gewonnen 5. Die Enzyme, die sich in dem Siebfiltrat befinden, werden anschließend ausgefällt 6, das Gemisch filtriert 7 und der Filterkuchen gewonnen 8. Der gewonnene Filterkuchen wird schließlich gemahlen 9, vakuumgetrocknet 10 und erneut gemahlen, wodurch das Pankreatin erhalten wird. Die mit den Bezugszeichen 2 bis 10 gekennzeichneten Verfahrensschritte führen jeweils zu Zwischenprodukten, die im Folgenden als Zwischenstufen bezeichnet werden.

Die Wirksubstanzen im Pankreatin sind u. a. verschiedene polymerabbauende Enzyme, wie Lipasen, Amylasen und Proteasen. Eine Voraussetzung für die Wirksamkeit des Therapeutikums ist, dass alle Enzyme in einem bestimmten Verhältnis und in aktiver Form im Wirkstoff vorhanden sind. Eine Besonderheit des Pankreatins ist, dass die enthaltenen Enzyme teilweise in Lösung und teilweise an Partikel gebunden vorliegen und es sich somit um eine Suspension handelt.

Untersuchungen zur Virusbelastung von Pankreatin haben gezeigt, dass behüllte, nicht aber unbehüllte Viren durch den aktuellen Produktionsprozess signifikant inaktiviert werden Grundsätzlich sollten pharmazeutische Wirkstoffe keine infektiösen Viren enthalten. Da die aktuellen Produktionsprozesse offenbar nicht in der Lage sind, eine potentiell vorhandene Kontamination von unbehüllten Viren mit ausreichender Sicherheitsmarge zu beseitigen, müssen zusätzliche virenreduzierende Schritte implementiert werden.

Klassische vireninaktivierende Methoden wie z. B. trockene oder feuchte Hitze oder virenabreichernde Methoden wie z. B. Filtration oder Chromatographie lassen sich bei Extrakten aus biologischem Ausgangsmaterial und insbesondere bei Organextrakten ohne Veränderung der Zusammensetzung und/oder hohe Produktverluste in den meisten Fällen nicht anwenden. Ein besonderes Problem dieser Extrakte sind häufig nicht gelöste Bestandteile, die ihnen eine suspensionsartige Eigenschaft verleihen. Dadurch kommt es zur Verblockung von Filtern oder Chromatographiesäulen. Weiterhin sind die wirksamen Substanzen oft sowohl in der gelösten als auch in der partikulären Fraktion verteilt und werden somit durch mechanische Abtrennverfahren teilweise entfernt.

Weitere Verfahren zur Inaktivierung von Viren, Bakterien und Pilzen in biologischen Materialien sind bekannt. Beispielsweise beschreibt WO 02/056824 A2 die Inaktivierung von Pathogenen in biologischen Materialien durch die Anwendung eines hohen Druckes. Die Erfindung bezieht sich auf die Behandlung von Blutplasma, also einer Lösung von biologischen aktiven Stoffen in Wasser. Bradley D. W. et al. beschreiben in "Pressure cycling technology: a novel approach to virus inactivation in plasma" (Transfusion, 40, 2000, 193) ebenfalls ein Verfahren zur Behandlung von Blutplasma.

Die Hochdruck-Behandlung von Lebensmitteln ist verschiedentlich beschrieben worden. Beispielsweise können Muscheln mit hohem Druck behandelt werden, um Noroviren oder Hepatitis-A-Viren zu inaktivieren (Kingsley et al., Inactivation of a Norovirus by High-Pressure Processing, Appl. Environ. Microbiol. 2007, 581; Calci et al., High-Pressure Inactivation of Hepatitis A Virus within Oysters, Appl. Environ. Microbiol. 2005, 339). Die Behandlung soll die organoleptischen Eigenschaften des Lebensmittels erhalten. Die biologische Aktivität von Enzymen oder anderen Wirkstoffen nach der Hochdruckbehandlung wurde nicht untersucht. Überdies wurden in beiden Fällen Feststoffe untersucht.

Es ist jedoch auch bekannt, dass nicht ohne weiteres vorhergesagt werden kann, ob mittels Hochdruckbehandlung tatsächlich eine Inaktivierung bestimmter Viren gelingt (Grove S. F. et al., Inactivation of Foodborne Viruses of Significance by High Pressure and other Processes, J. Food Prot. 2006, 667). Je nach dem, ob es sich um flüssige Proben oder feste Proben handelt, müssen aufgrund der unterschiedlichen Kompressibilität der Proben unterschiedliche Verfahrensbedingungen gewählt werden.

Außerdem kann die Hochdruckbehandlung zu einer Veränderung der Lebensmittel führen. Beispielsweise werden Früchte braun, wenn sie einer solchen Behandlung unterzogen werden. Diese Farbänderung ist auf eine enzymatische Reaktion zurückzuführen, was auf eine Veränderung der biologischen Aktivität dieser Enzyme schließen lässt.

Eine besondere Schwierigkeit besteht bei biologischen Proben, die nicht in weitgehend homogener Form vorliegen. Bei einem feststoffhaltigen Extrakt in Form einer Suspension befinden sich Teile der biologisch aktiven Wirkstoffe in der flüssigen Phase, andere Teile in den dispergierten Feststoffteilchen. Eine Inaktivierung von Viren in den Feststoffteilen kann daher mit einer Zerstörung der Wirkstoffe verbunden sein, die sich in der flüssigen Phase befinden, da die Kompressibilität der flüssigen Phase höher als die der Feststoffteilchen ist.

Pankreatin oder die bei der Herstellung anfallenden Zwischenstufen stellen aufgrund ihrer natürlichen Virusbelastung und ihres Suspensionscharakters beispielhafte feststoffhaltige biologische Extrakte dar. Bei klassischen Spiking-Experimenten wird die entsprechende Substanz mit verschiedenen Laborstämmen gespikt und der Titer vor und nach der Behandlung bestimmt.

Es besteht darüber hinaus ein Bedarf an Verfahren, bei denen die in einem feststoffhaltigen biologischen Extrakt enthaltenen viralen und bakteriellen Belastungen vollständig oder auf ein Minimum reduziert werden. Das Verfahren muss gleichermaßen für Feststoffe und Suspensionen geeignet sein. Das Verfahren soll insbesondere die Inaktivierung von unbehüllten Viren im Pankreatin ermöglichen.

Aufgabe der Erfindung ist es, die Nachteile nach dem Stand der Technik zu beseitigen. Es soll insbesondere ein Verfahren zur Verringerung der viralen und mikrobiellen Belastung feststoffhaltiger biologischer Extrakte angegeben werden, das für Feststoffe und Suspensionen geeignet ist, die Aktivität der in dem biologischen Extrakt enthaltenen Enzyme nicht wesentlich verringert, die pharmakologisch gewünschten Eigenschaften nicht verschlechtert und keine toxischen chemischen Verbindungen erzeugt. Ferner sollen mittels des Verfahrens hergestellte Extrakte und Verwendungen derartiger Extrakte angegeben werden.

Diese Aufgabe wird durch die Merkmale der Ansprüche 1, 16, 18 und 19 gelöst, Zweckmäßige Ausgestaltungen der Erfindung ergeben sich aus den Merkmalen der Ansprüche 2 bis 15 sowie 17.

Nach Maßgabe der Erfindung ist ein Verfahren zur Verringerung der viralen und mikrobiellen Belastung feststoffhaltiger biologischer Extrakte vorgesehen, umfassend die Schritte
(a) Bereitstellen des feststoffhaltigen biologischen Extraktes, umfassend zumindest einen biologisch aktiven Wirkstoff, ausgewählt aus Enzymen, Proteinen und Peptiden, oder ein Gemisch derartiger Wirkstoffe; und
(b) Unterziehen des in Schritt (a) bereitgestellten Extraktes einer Hochdruckbehandlung;
wobei
- die biologische Aktivität des feststoffhaltigen biologischen Extraktes nach der Hochdruckbehandlung zumindest 50 % der biologischen Aktivität des feststoffhaltigen biologischen Extraktes vor der Hochdruckbehandlung entspricht; und
- die virale Infektiosität des feststoffhaltigen biologischen Extraktes nach der Hochdruckbehandlung um einen Faktor von größer als 1 log₁₀ im Vergleich zur viralen Infektiosität vor der Hochdruckbehandlung verringert worden ist.

Vorzugsweise wird der feststoffhaltige biologische Extrakt als Suspension, umfassend eine flüssigen Phase und darin dispergierte Feststoffteilchen, bereitgestellt, wobei das Gemisch biologisch aktiver Wirkstoffe zu einem Teil in der flüssigen Phase gelöst und zu einem anderen Teil an die Feststoffteilchen gebunden ist. Ebenso bevorzugt kann der feststoffhaltige biologische Extrakt in fester Form bereitgestellt werden.

Unter dem Begriff "feststoffhaltiger biologischer Extrakt" soll hier ein Extrakt verstanden werden, der einen biologisch aktiven Wirkstoff, ausgewählt aus Enzymen, Proteinen und Peptiden, oder ein Gemisch derartiger Wirkstoffe umfasst. Es ist bevorzugt, dass der feststoffhaltige biologische Extrakt ein Gemisch der genannten biologisch aktiven Wirkstoffe umfasst. Im Folgenden wird der feststoffhaltige biologische Extrakt der Einfachheit halber auch als Extrakt bezeichnet.

Der feststoffhaltige biologische Extrakt ist vorzugsweise ein Extrakt, der als alkoholischer oder wässeriger Extrakt aus tierischen Organen gewonnen wurde. Der enthaltene biologisch aktive Wirkstoff oder das Wirkstoffgemisch können in dem Extrakt sowohl in Lösung als auch an Feststoffe gebunden vorliegen. Ein solches feststoffhaltiges biologisches Extrakt ist ein komplexer Extrakt. Der biologisch aktive Wirkstoff oder das Gemisch derartiger Wirkstoffe weist vorzugsweise pharmazeutische Aktivität auf.

Unter dem Begriff "biologisch aktiver Wirkstoff" werden pharmazeutische Wirkstoffe und therapeutische Wirkstoffe verstanden. Biologisch aktive Wirkstoffe im Sinne dieser Erfindung sind beispielsweise Enzyme, Proteine und Peptide.

Beispielhafte tierische Organe, aus dem der Extrakt gewonnen werden kann, sind die Leber, der Pankreas und die Magenschleimhaut.

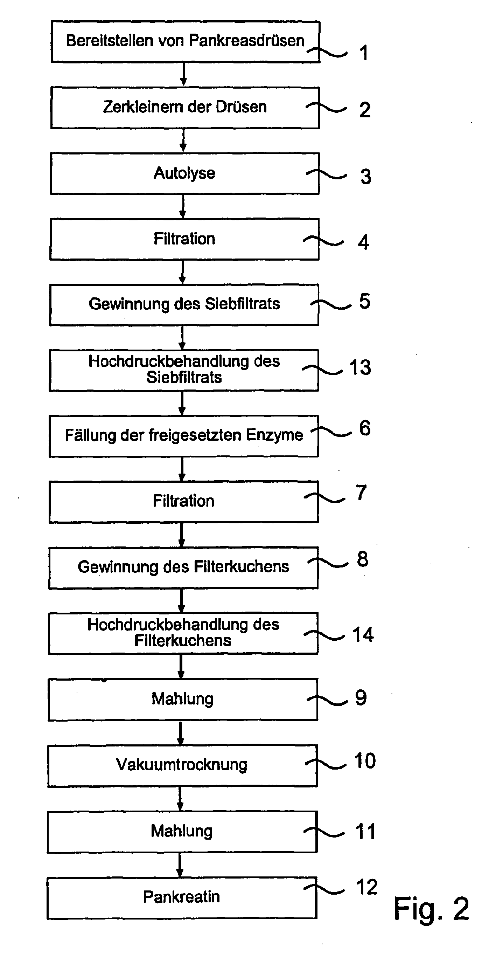

Beispiele erfindungsgemäßer feststoffhaltiger biologischer Extrakte sind Extrakte, die aus dem Pankreas, insbesondere dem Pankreas des Schweins, gewonnen werden. Spezielle Beispiele sind das Pankreatin sowie die bei dessen Herstellung anfallenden Zwischenstufen. Diese Zwischenstufen sind in Fig. 2 mit den Bezugszeichen 2 bis 10 gekennzeichnet. Bevorzugte Beispiele dieser Zwischenstufen sind das Siebfiltrat (Bezugszeichen 5 in Fig. 2) und der Filterkuchen (Bezugszeichen 8 in Fig. 2), nach deren Gewinnung eine Hochdruckbehandlung durchgeführt werden kann, d. h. das Siebfiltrat und/oder der Filterkuchen können einer Hochdruckbehandlung unterzogen werden (Bezugszeichen 13 bzw. 14 in Fig. 2). Zusätzlich oder alternativ kann eine Hochdruckbehandlung auch mit jeder der Zwischenstufen mit den Bezugszeichen 2, 3, 4, 6, 7, 9, 10 und 11 durchgeführt werden.

Aus dem Pankreas wird Pankreatin als pharmazeutischer Wirkstoff gewonnen. Pankreatin sowie die bei der Herstellung anfallende Zwischenstufen enthalten u. a. die Enzyme Lipase, Amylase und Protease. Sie sind somit feststoffhaltige biologische Extrakte im Sinne der vorliegenden Erfindung. Die Extrakte werden vorzugsweise als wässerig-alkoholische Extrakte in Schritt (a) des erfindungsgemäßen Verfahrens bereitgestellt.

Das erfindungsgemäße Verfahren ist auf alle Virusformen, wie DNA- und RNA-Viren, behüllte und unbehüllte Viren, weiterhin auf Virionen und Prionen oder ähnliche biologische Systeme sowie Bakterien und Pilze anwendbar. Das Verfahren wird bevorzugt zur Verringerung der Belastung von unbehüllten Viren im Pankreatin aus dem Schweine-Pankreas oder in entsprechenden bei der Herstellung anfallenden Zwischenstufen (siehe Fig. 2) verwendet.

Das erfindungsgemäße Verfahren erlaubt die Reduktion der Viren- und Mikroorganismen-Belastung von feststoffhaltigen biologischen Extrakten, ohne dass sich deren enzymatische Aktivität wesentlich verringert, die pharmakologisch beabsichtigten Eigenschaften verschlechtern oder toxische chemische Verbindungen erzeugt werden.

Die biologische Aktivität des feststoffhaltigen biologischen Extraktes nach der Hochdruckbehandlung sollte zumindest 50 % der biologischen Aktivität des feststoffhaltigen biologischen Extraktes vor der Hochdruckbehandlung betragen, bevorzugt zumindest 80 %, besonders bevorzugt zumindest 90 %.

Ist zumindest einer der biologisch aktiven Wirkstoffe, die in dem Extrakt enthalten sind, ein Enzym, so sollte die enzymatische Aktivität des feststoffhaltigen biologischen Extraktes nach der Hochdruckbehandlung zumindest 50 % der enzymatischen Aktivität des feststoffhaltigen biologischen Extraktes vor der Hochdruckbehandlung betragen, bevorzugt zumindest 80 %, besonders bevorzugt zumindest 90 %.

In Bezug auf Pankreatin sollten dessen Lipase-Aktivität, dessen Amylase-Aktivität und/oder dessen Protease-Aktivität nach der Hochdruckbehandlung wenigstens 50 %, bevorzugt 80 %, besonderes bevorzugt 90 % seiner Lipase-Aktivität, der Amylase-Aktivität und/oder der Protease-Aktivität vor der Hochdruckbehandlung betragen.

Die virale Infektiosität des feststoffhaltigen biologischen Extraktes nach der Hochdruckbehandlung sollte um einen Faktor von größer als 1 log₁₀, vorzugsweise größer 3 log₁₀, besonders bevorzugt größer 4 log₁₀, im Vergleich zur viralen Infektiosität vor der Hochdruckbehandlung verringert worden sein. Dies gilt insbesondere auch für die virale Infektiosität von unbehüllten Viren. Beispielsweise sollte die Infektiosität von unbehüllten Viren im Pankreatin nach der Behandlung um einen Faktor von größer als 1 log₁₀, vorzugsweise größer 3 log₁₀, besonders bevorzugt größer 4 log₁₀, im Vergleich zu dessen Infektiosität vor der Behandlung verringert sein.

Die virale Infektiosität wird durch Endpunkttitration und anschließende Berechnung des Halbwertes der Gewebekultur-Infektionsdosis (TCID₅₀) gemäß der Spearman-Kärber-Formel berechnet (Bundesanzeiger, Nr. 84, 4. Mai 1994). Die so errechneten Virustiter werden als log₁₀ TCID₅₀ pro ml mit Konfidenzintervallen von 95 % angegeben.

Die Reduktion der viralen Belastung wird gemäß der EG-Richtlinie CMMP/BWP/268/95, Anhang II) als logarithmischer Reduktionsfaktor angegeben, der die Differenz der Virustiter zwischen einer Kontrollprobe und den druckbehandelten Proben ist.

Die enzymatischen Aktivitäten von Pankreatin werden gemäß der Monografie "Pancreas powder", veröffentlicht in der Pharmacopoea Europaea 6.2, 01/2008:0350, bestimmt.

Bei der Hochdruckbehandlung des Extraktes werden keine chemischen Substanzen zugesetzt, so dass die Belastung mit toxischen Substanzen nach der Behandlung nicht höher als vor der Behandlung ist.

Schritt (b) des erfindungsgemäßen Verfahrens wird vorzugsweise unter Anwendung von Drücken im Bereich von 1000 bis 8000 bar durchgeführt. Bevorzugt im Bereich von 2000 bis 7000 bar, besonders bevorzugt im Bereich von 3000 bis 6000 bar. Die Hochdruckbehandlung kann erfindungsgemäß bei konstantem Druck oder durch Druckimpulse erfolgen. Die Hochdruckbehandlung kann im Durchflussverfahren oder im Batchverfahren in entsprechenden Apparaturen stattfinden. Die Einwirkzeit der Hochdruckbehandlung auf das in Schritt (a) bereitgestellte Extrakt beträgt vorzugsweise 1 bis 60 min. Die Hochdruckbehandlung wird bevorzugt bei Temperaturen zwischen -20 °C und 30 °C, besonders bevorzugt zwischen -10 °C und 20 °C durchgeführt.

Die flüssige Phase des Extraktes, der einer Hochdruckbehandlung in Schritt (b) unterzogen wird, kann als Lösungsmittel Wasser oder ein Gemisch aus Wasser und einem organischen Lösungsmittel enthalten. Der Lösungsmittel-Anteil kann im Bereich zwischen 1 und 90 Gew.-%, bezogen auf das Gewicht der Suspension, liegen. Im Fall der bei der Pankreatinherstellung anfallenden biologischen Extrakte ist das verwendete Lösungsmittel vorzugsweise ein Alkohol, besonders bevorzugt Isopropanol. Der Isopropanol-Anteil liegt typischerweise zwischen 5 und 85 Gew.-%, bezogen auf das Gewicht der Suspension.

Nach Maßgabe der Erfindung ist ferner ein feststoffhaltiger biologischer Extrakt vorgesehen, der durch das erfindungsgemäße Verfahren erhalten wurde. Dieser Extrakt ist durch geringe virale und mikrobielle Belastung gekennzeichnet. Trotz der vorherigen Hochdruckbehandlung ist seine biologische, insbesondere seine enzymatische Aktivität nicht wesentlich verringert, sind seine pharmakologisch beabsichtigen Eigenschaften nicht verschlechtert, und er weist keine toxischen chemischen Verbindungen auf, die bei der Behandlung zugegeben wurden.

Der erfindungsgemäße, durch Hochdruckbehandlung erhaltene Extrakt bzw. der daraus hergestellte biologische Wirkstoff kann für die Herstellung von Arzneimitteln, insbesondere im Rahmen der oralen Enzymtherapie, sowie als Nahrungs- oder Lebensmittel oder Nahrungsergänzungsmittel verwendet werden.

Die Erfindung wird nachfolgend anhand von Beispielen, die die Erfindung nicht beschränken sollen, unter Bezugnahme auf die Zeichnungen näher erläutert. Dabei zeigen
- Fig. 1: ein Ablaufschema eines Verfahrens zur Herstellung von Pankreatin aus Schweine-Pankreasdrüsen nach dem Stand der Technik;
- Fig. 2: ein Ablaufschema eines erfindungsgemäßen Verfahrens zur Herstellung von Pankreatin aus Schweine-Pankreasdrüsen;

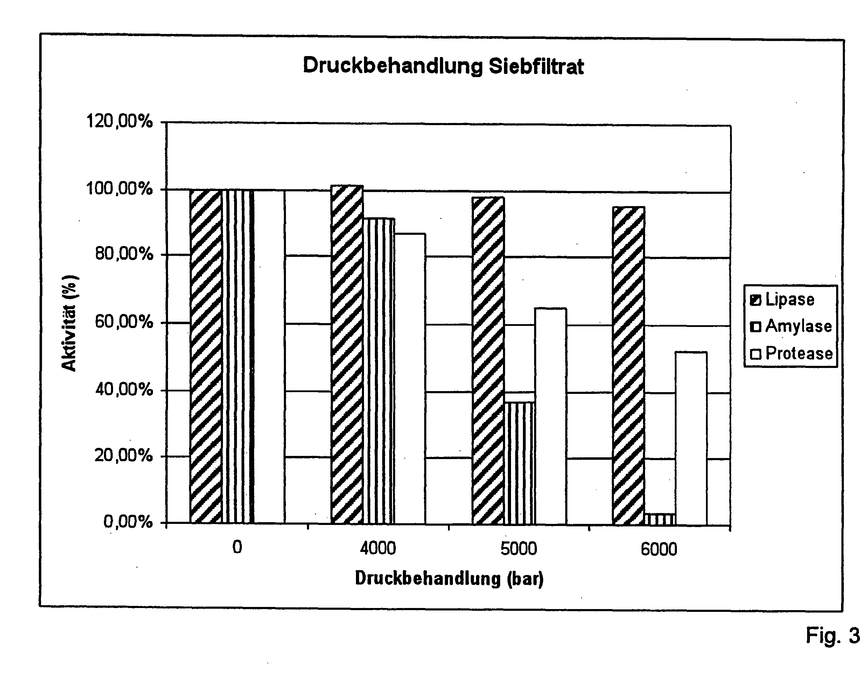

- Fig. 3: ein Diagramm, das die relativen enzymatischen Aktivitäten nach Behandlung von Siebfiltrat aus dem Pankreatin- Produktionsprozess mit unterschiedlichen Drücken in Pro- zent, bezogen auf die Ausgangsaktivität der unbehandelten Probe, zeigt; und

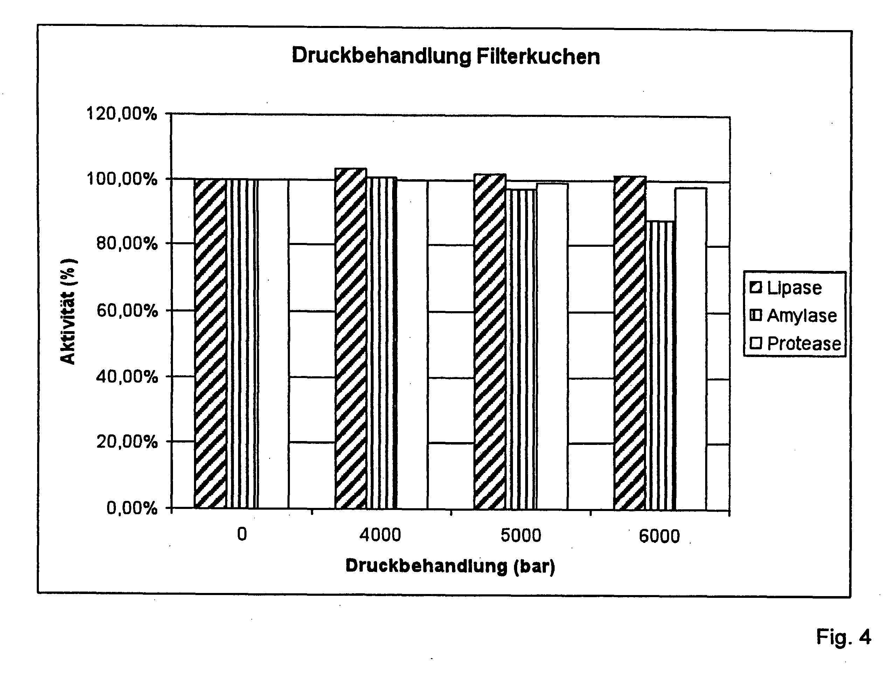

- Fig. 4: ein Diagramm, das die relativen enzymatischen Aktivitäten nach Behandlung von Filterkuchen aus dem Pankreatin- Produktionsprozess mit unterschiedlichen Drücken in Pro- zent, bezogen auf die Ausgangsaktivität der unbehandelten Probe, zeigt.

Eine Ausführungsform des erfindungsgemäßen Verfahrens zur Herstellung von Pankreatin wird nachstehend unter Bezugnahme auf Fig. 2 beschrieben. Die von Hausschweinen stammenden Pankreasdrüsen 1 werden zunächst zerkleinert 2 und einer Autolyse 3 unterzogen. Durch Filtration 4 des so erhaltenen Zwischenproduktes wird das Siebfiltrat gewonnen 5. Das Siebfiltrat kann vor der Weiterbehandlung einer Hochdruckbehandlung unterzogen werden 13. Anschließend werden die Enzyme, die sich in dem Siebfiltrat befinden, ausgefällt 6, das Gemisch filtriert 7 und der Filterkuchen gewonnen 8. Der gewonnene Filterkuchen kann einer Hochdruckbehandlung unterzogen werden 14. Der Filterkuchen wird schließlich gemahlen 9, vakuumgetrocknet 10 und erneut gemahlen, wodurch das Pankreatin erhalten wird 12.

Gemäß dem in Fig. 2 dargestellten Verfahren kann (i) eine Hochdruckbehandlung des Siebfiltrates 13 oder (ii) eine Hochdruckbehandlung des Filterkuchens 14 oder (iii) eine Hochdruckbehandlung des Siebfiltrates 13 und eine Hochdruckbehandlung des Filterkuchens 14 vorgesehen sein. Darüber hinaus kann die Hochdruckbehandlung auch an allen anderen Intermediaten des Produktionsprozesses durchgeführt werden.

### Beispiel 1

Das folgende Beispiel beschreibt die Behandlung von Zwischenstufen aus dem Pankreatin-Herstellungsverfahren als feststoffhaltige biologische Extrakte, die aus dem Schweine-Pankreas gewonnen wurden, mit Hochdruck.

### (1) Durchführung

Die Behandlung wurde im Batchverfahren in einer Hochdruckapparatur durchgeführt.
a) Eine in 40%igem Isopropanol gelöste Zwischenstufe des Pankreatin-Produktionsprozesses (Siebfiltrat mit ca. 5 Gew.-% Feststoffanteil, bezogen auf das Gewicht des Siebfiltrats, siehe Bezugszeichen 13 von Fig. 2) wurde für 5 min bei 15 °C einer Hochdruckbehandlung bei 4000, 5000 und 6000 bar unterzogen.
b) Alternativ wurde unter den gleichen Bedingungen eine Hochdruckbehandlung mit Filterkuchen (siehe Bezugszeichen 14 von Fig. 2, Feststoffanteil ca. 50 Gew.-%, Flüssiganteil, bestehend zu ca. 80 Gew.-% aus Isopropanol und zu ca. 20 Gew.-% aus Wasser) durchgeführt.

Für beide Proben wurde die Aktivität von Lipase, Amylase und Protease im Vergleich zu einer unbehandelten Probe bestimmt.

### (2) Bewertung

Die dargestellten Ergebnisse zeigen, dass die drei gemessenen Enzymaktivitäten im Filterkuchen (Fig. 2) auch bei einer Hochdruckbehandlung mit 6000 bar nicht signifikant abnehmen. Die Lipaseaktivität war auch im Siebfiltrat (Fig. 2) bei allen Drücken stabil. Im Gegensatz dazu nahm die Amylaseaktivität im Siebfiltrat bei Drücken > 4000 bar deutlich ab.

### Beispiel 2

Das folgende Beispiel beschreibt die Inaktivierung des unbehüllten Virus FCV mittels Hochdruck (FCV = Felines Calicivirus).

### (1) Durchführung

Die Behandlung wurde im Batchverfahren in einer Hochdruckapparatur durchgeführt.

Eine FCV-Stocklösung in Kulturmedium wurde bei unterschiedlichen Drücken und Temperaturen für unterschiedliche Zeiten einer Hochdruckbehandlung unterzogen. Der Virustiter wurde in der Ausgangsprobe und in den behandelten Proben bestimmt.

### (2) Bewertung

Die dargestellten Ergebnisse (Tab. 1) zeigen, dass der Virustiter durch die Hochdruckbehandlung bei allen Proben, außer bei der Probe, die bei 20 °C, 5 min mit 4000 bar behandelt worden war, bis unter die Nachweisgrenze von 1,9 log₁₀ sinkt. Aus der Differenz der Virustiter zwischen unbehandelter Probe und behandelten Proben ergibt sich ein Abreicherungsfaktor von ≥ 6,3 log₁₀. FCV wird als Modellvirus für den ebenfalls unbehüllten HEV verwendet, der bei Schweinen nachgewiesen wurde und als human pathogen gilt, aber selber nicht in Zellkultur nachgewiesen werden kann. Eine Abreicherung dieses Virus um > 6 log₁₀ Stufen, stellt eine wesentliche Verbesserung der biologischen Sicherheit von Pankreatin dar.

**Tabelle 1: Virustiter (TCDI₅₀ in log₁₀) nach Hochdruckbehandlung einer FCV Lösung**

| **Behandlung** | | **4000 bar** | **5000 bar** | **6000 bar** |
|---|---|---|---|---|
| **Zeit (min)** | **Temperatur (°C)** | | | |
| 0 | 20 | 8,2 | | |
| 5 | 20 | 3,6 | < 1,9 | < 1,9 |
| 5 | 0 | < 1,9 | < 1,9 | < 1,9 |
| 5 | -5 | < 1,9 | < 1,9 | < 1,9 |
| 15 | 0 | < 1,9 | < 1,9 | < 1,9 |

## Patentansprüche

1. Verfahren zur Verringerung der viralen und mikrobiellen Belastung feststoffhaltiger biologischer Extrakte, umfassend die Schritte
(a) Bereitstellen des feststoffhaltigen biologischen Extraktes, umfassend zumindest einen biologisch aktiven Wirkstoff, ausgewählt aus Enzymen, Proteinen und Peptiden, oder ein Gemisch derartiger Wirkstoffe; und
(b) Unterziehen des in Schritt (a) bereitgestellten Extraktes einer Hochdruckbehandlung;
wobei
- die biologische Aktivität des feststoffhaltigen biologischen Extraktes nach der Hochdruckbehandlung zumindest 50 % der biologischen Aktivität des feststoffhaltigen biologischen Extraktes vor der Hochdruckbehandlung entspricht, und
- die virale Infektiosität des feststoffhaltigen biologischen Extraktes nach der Hochdruckbehandlung um einen Faktor von größer als 1 log₁₀ im Vergleich zur viralen Infektiosität vor der Hochdruckbehandlung verringert worden ist.

2. Verfahren nach Anspruch 1,
**dadurch gekennzeichnet,**
**dass** der feststoffhaltige biologische Extrakt als Suspension, umfassend eine flüssigen Phase und darin dispergierte Feststoffteilchen, bereitgestellt wird, wobei das Gemisch biologisch aktiver Wirkstoffe zu einem Teil in der flüssigen Phase gelöst und zu einem anderen Teil an die Feststoffteilchen gebunden ist.

3. Verfahren nach Anspruch 1,
**dadurch gekennzeichnet,**
**dass** der feststoffhaltige biologische Extrakt in fester Form bereitgestellt wird.

4. Verfahren nach einem der vorstehenden Ansprüche,
**dadurch gekennzeichnet,**
**dass** der feststoffhaltige biologische Extrakt aus einem tierischen Organ gewonnen wurde.

5. Verfahren nach einem der vorstehenden Ansprüche,
**dadurch gekennzeichnet,**
**dass** der feststoffhaltige Extrakt ein Extrakt aus dem Pankreas des Schweins ist.

6. Verfahren nach einem der vorstehenden Ansprüche
**dadurch gekennzeichnet,**
**dass** die Hochdruckbehandlung bei einem Druck von 1000 bis 8000 bar durchgeführt wird.

7. Verfahren nach einem der vorstehenden Ansprüche,
**dadurch gekennzeichnet,**
**dass** die Hochdruckbehandlung bei einem konstanten Druck oder mit pulsierenden Drücken durchgeführt wird.

8. Verfahren nach einem der vorstehenden Ansprüche,
**dadurch gekennzeichnet,**
**dass** der feststoffhaltige biologische Extrakt vor der Hochdruckbehandlung einem Filtrationsschritt unterzogen wird, wodurch ein Filtrat und ein Filterkuchen erhalten werden, wobei der Filterkuchen der Hochdruckbehandlung unterzogen wird.

9. Verfahren nach Anspruch 8,
**dadurch gekennzeichnet,**
**dass** der Filterkuchen einen Feststoffanteil von zumindest 40 Gew.-%, bezogen auf das Gewicht des Filterkuchens, aufweist.

10. Verfahren nach einem der vorstehenden Ansprüche,
**dadurch gekennzeichnet,**
**dass** die biologische Aktivität des feststoffhaltigen biologischen Extraktes nach der Hochdruckbehandlung zumindest 90 % der biologischen Aktivität des feststoffhaltigen biologischen Extraktes vor der Hochdruckbehandlung entspricht.

11. Verfahren nach einem der vorstehenden Ansprüche,
**dadurch gekennzeichnet,**
**dass**, sofern zumindest einer der biologisch aktiven Wirkstoffe ein Enzym ist, die enzymatische Aktivität des feststoffhaltigen biologischen Extraktes nach der Hochdruckbehandlung zumindest 50 % der enzymatischen Aktivität des feststoffhaltigen biologischen Extraktes vor der Hochdruckbehandlung entspricht.

12. Verfahren nach einem der vorstehenden Ansprüche,
**dadurch gekennzeichnet,**
**dass**, sofern zumindest einer der biologisch aktiven Wirkstoffe ein Enzym ist, die enzymatische Aktivität des feststoffhaltigen biologischen Extraktes nach der Hochdruckbehandlung zumindest 90 % der enzymatischen Aktivität des feststoffhaltigen biologischen Extraktes vor der Hochdruckbehandlung entspricht.

13. Verfahren nach einem der vorstehenden Ansprüche,
**dadurch gekennzeichnet,**
**dass** die virale Infektiosität des feststoffhaltigen biologischen Extraktes nach der Hochdruckbehandlung um einen Faktor von größer als 3 log₁₀ im Vergleich zur viralen Infektiosität vor der Hochdruckbehandlung verringert worden ist.

14. Verfahren nach einem der vorstehenden Ansprüche,
**dadurch gekennzeichnet,**
**dass** die virale Infektiosität des feststoffhaltigen biologischen Extraktes nach der Hochdruckbehandlung um einen Faktor von größer als 4 log₁₀ im Vergleich zur viralen Infektiosität vor der Hochdruckbehandlung verringert worden ist.

15. Verfahren nach einem der Ansprüche,
**dadurch gekennzeichnet,**
**dass** die Belastung des feststoffhaltigen biologischen Extraktes mit toxischen Substanzen nach der Hochdruckbehandlung nicht höher als vor der Hochdruckbehandlung ist.

16. Feststoffhaltiger biologischer Extrakt, erhalten durch das Verfahren nach einem der Ansprüche 1 bis 15, wobei
- die biologische Aktivität des feststoffhaltigen biologischen Extraktes zumindest 50 % der biologischen Aktivität des in Schritt (a) bereitgestellten feststoffhaltigen biologischen Extraktes entspricht, der keiner Hochdruckbehandlung unterzogen wurde; und
- die virale Infektiosität des feststoffhaltigen biologischen Extraktes um einen Faktor von größer als 1 log₁₀ im Vergleich zur viralen Infektiosität des in Schritt (a) bereitgestellten feststoffhaltigen biologischen Extraktes verringert worden ist.

17. Feststoffhaltiger biologischer Extrakt nach Anspruch 16,
**dadurch gekennzeichnet,**
**dass** der Extrakt ein Extrakt aus dem Pankreas des Schweins ist.

18. Verwendung eines feststoffhaltigen biologischen Extraktes nach einem der Ansprüche 17 oder 18 zur Herstellung eines Arzneimittels.

19. Verwendung eines feststoffhaltigen biologischen Extraktes nach einem der Ansprüche 17 oder 18 als Nahrungs- oder Lebensmittel oder als Nahrungsergänzungsmittel
